(19) Europäisches Patentamt
European Patent Office
Office européen des brevets

(11) EP 3 551 637 B1

(12) **EUROPEAN PATENT SPECIFICATION**

(45) Date of publication and mention of the grant of the patent:
**04.11.2020 Bulletin 2020/45**

(21) Application number: **17829044.1**

(22) Date of filing: **05.12.2017**

(51) Int Cl.:
***C07F 9/6561*** (2006.01) ***A61K 31/675*** (2006.01)
***A61K 49/00*** (2006.01) ***C09K 11/07*** (2006.01)
***G01N 21/64*** (2006.01)

(86) International application number:
**PCT/IN2017/050571**

(87) International publication number:
**WO 2018/104963 (14.06.2018 Gazette 2018/24)**

(54) **A COMPOUND FOR THE DETECTION OF HNO IN BIOLOGICAL SYSTEMS**

VERBINDUNG ZUM NACHWEIS VON HNO IN BIOLOGISCHEN SYSTEMEN

COMPOSÉ POUR LA DÉTECTION DE HNO DANS DES SYSTÈMES BIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.12.2016 IN 201611041925**

(43) Date of publication of application:
**16.10.2019 Bulletin 2019/42**

(73) Proprietor: **Council of Scientific and Industrial Research**
**New Delhi 110001 (IN)**

(72) Inventors:
- **DAS, Amitava**
  **Pune 411 008**
  **Maharashtra (IN)**
- **ALI, Firoj**
  **Pune 411 008**
  **Maharashtra (IN)**
- **ASHOK, Anila Hoskere**
  **Pune 411 008**
  **Maharashtra (IN)**
- **CHATTOPADHYAY, Samit**
  **Ganeshkhind 411 007**
  **Maharashtra (IN)**
- **TAYE, Nandaraj**
  **Ganeshkhind 411 007**
  **Maharashtra (IN)**

(74) Representative: **Manuel Illescas y Asociados, S.L.**
**C/ Príncipe de Vergara 197, Oficina 1°A**
**28002 Madrid (ES)**

(56) References cited:
- **LIU PING ET AL: "A Near-Infrared Fluorescent Probe for Detection of Nitroxyl in Living Cells", CHINESE JOURNAL OF ANALYTICAL CHEMISTRY, vol. 43, no. 12, December 2015 (2015-12), pages 1829-1836, XP029383288, ISSN: 1872-2040, DOI: 10.1016/S1872-2040(15)60883-0**
- **FIROJ ALI ET AL: "A fluorescent probe for specific detection of cysteine in the lipid dense region of cells", CHEMICAL COMMUNICATIONS, vol. 51, no. 95, 1 January 2015 (2015-01-01), pages 16932-16935, XP055312761, ISSN: 1359-7345, DOI: 10.1039/C5CC07450A cited in the application**
- **FIROJ ALI ET AL: "A Super-Resolution Probe To Monitor HNO Levels in the Endoplasmic Reticulum of Cells", ANALYTICAL CHEMISTRY, vol. 89, no. 22, 7 November 2017 (2017-11-07), pages 12087-12093, XP055447802, US ISSN: 0003-2700, DOI: 10.1021/acs.analchem.7b02567**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION:

The present invention relates to a novel compound for detecting HNO in biological systems. More particularly, the present invention relates to a compound of Formula (I), process for preparation thereof and such a compound of Formula (I) for use in detecting HNO in biological systems. The present invention further relates to a kit comprising compound of Formula (I).

### BACKGROUND AND PRIOR ART OF THE INVENTION:

Nitroxyl (HNO) is an elusive chemical species that has been shown to possess intriguing biological properties. For example, nitroxyl has been implicated in the mechanism of cyanamide's inhibitory effect on aldehyde dehydrogenase in treating alcohol abuse as well as reversing experimental heart failure.

Despite HNO having been described in the chemical literature for decades, there are surprising gaps in the literature that complicate the rational exploitation of its pharmacological properties. A number of studies have suggested that it may confer important biological functions in various physiological processes. For example, HNO also known to interact with bio-thiols in aldehyde dehydrogenase, leading to the inhibition of the enzyme's activity. HNO could mediate relaxation of resistant-like arteries by activating voltage-dependent K+ channel. Also, nitroxyl (HNO) is involved in the regulating cardiovascular functions and may provide useful tools for treating cardiovascular diseases such as heart failure. The role of HNO in different biological processes still remains largely unknown. HNO is a new candidate heart failure drug therapy, has been shown to enhance overall cardiovascular function in both healthy and failing hearts, at least in part, by increasing $Ca^{2+}$ re-uptake into the CSR and SERCA2a, that regulates intracellular $Ca^{2+}$- handling and thus plays a critical role in initiating cardiac contraction and relaxation. HNO can increase systolic force and decrease diastolic pressure in both normal and failing canine hearts through upregulating the calcitonin gene-related peptide. Thus, more and more observations suggest that HNO may provide a powerful therapeutic agent for heart failure cure.

Several methodologies based on various analytical techniques (e.g., colorimetric methods, EPR, HPLC, mass spectrometry and electrochemical analysis) for the detection of HNO are available in the literature. Such methodologies are either time-consuming or involve the destruction of cells and tissues and are thus not ideally suited to *in vivo* tracking and detection of HNO. In this context, reagents that show fluorescence ON response on detection of HNO are used as an imaging reagent as well as for studying biospecies in living samples. Such reagents also allow high sensitivity and spatiotemporal resolution. Among the various reagents that show such a response, phosphine-based reagents have received considerable attention. King and co-workers were first to report the reaction of HNO with organic phosphines to produce the corresponding phosphine oxide and azaylide [J. Am. Chem. Soc., 2011, 133 (30), pp 11675-11685]. Since then, chemodosimetric probe molecules have been exploited in this reaction for specific detection of HNO using fluorophores like rhodamine, coumarine, napthalimide, and BODIPY. This reaction has also been exploited in developing FRET-based receptors for HNO. Most of these receptors show a luminescence response within the visible region of the spectrum.

Article titled "Development of green to near-infrared turn-on fluorescent probes for the multicolour imaging of nitroxyl in living systems" by B Dong published in J. Mater. Chem. B, 2016, 4, 1263-1269 reports three novel turn-on probes (NP-1-3) based on structurally related dyes with different emission colors as fluorescent scaffolds for detecting HNO in biological systems. The probes exhibit high sensitivity, excellent selectivity, desirable performance at physiological pH and low cytotoxicity.

Article titled "A fluorescent probe for specific detection of cysteine in the lipid dense region of cells" by Firoj Ali et al. published in Chem. Commun., 2015, 51, 16932-16935 reports a new cysteine (Cys) specific chemodosimetric reagent (ER-F) is used in imaging of endogenous Cys localized in the lipid dense region of the live Hct116 cells and the release of Cys within HepG2 cells from a drug following a biochemical transformation.

Article titled "A Near-Infrared Fluorescent Probe for Detection of Nitroxyl in Living Cells" by LIU Ping et al. published in Chinese Journal of Analytical Chemistry; 2015, 43 (12), pp 1829-1836 reports a near-infrared (NIR) metal-free fluorescent probe, ER-JN, designed and synthesized for the detection of intracellular HNO level in simulated physiological conditions and living cells. The as-prepared probe exhibited high sensitivity, good selectivity and low cytotoxicity and applied to fluorescent bio-imaging of HNO in simulated physiological conditions. When used in detection of HNO, quantum yield of the probe increased from 0.01 to 0.35. The linear range was 0.50 $\mu$M, with the detection limit of 0.03 $\mu$M ($S/N$ = 3).

Article titled "Resorufin based fluorescence 'turn-on' chemodosimeter probe for nitroxyl (HNO) " by KN Bobba et al. published in RSC Adv., 2015,5, 84543-84546 reports a cellular responsive, highly selective fluorogenic and chromogenic chemodosimeter probe for HNO. This new probe showed ~30 fold fluorescence enhancement in the presence of HNO and is sensitive to HNO at concentrations as low as 0.02 $\mu$M. Further, it is capable of detecting HNO levels in

cellular milieus as well as in live specimens *e.g. C. elegans.*

Article titled "A near-infrared fluorescent probe for the selective detection of HNO in living cells and in vivo" by P Liu et al. published in Analyst, 2015,140,pp 4576-4583 reports a near-infrared (NIR) metal-free fluorescent probe Cyto-JN for the detection of nitroxyl (HNO) in living cells and *in vivo.* The metal-free Cyto-JN is composed of two moieties: the Aza-BODIPY fluorophore and the HNO recognition unit, the diphenylphosphinobenzoyl group. Cyto-JN can react with HNO in a 1:1 stoichiometry, which may bring great benefit to the detection efficiency of bioassays.

Article titled "A highly sensitive and reductant-resistant fluorescent probe for nitroxyl in aqueous solution and serum" by GJ Mao et al. published in Chem. Commun., 2014,50, 5790-5792 reports a novel coumarin-based fluorescent probe, P-CM, for quantitative detection of nitroxyl (HNO). P-CM exhibits a selective response to HNO over other biological reductants and was also applied for quantitative detection of HNO in bovine serum with satisfactory results.

Article titled "A two-photon fluorescent turn-on probe for nitroxyl (HNO) and its bioimaging application in living tissues" by K Zheng et al. published in Chem. Commun., 2015, 51, 5754-5757 reports synthesis of two-photon fluorescent probe for specific detection of nitroxyl. They have demonstrated that the probe GCTPOC-1 is suitable for fluorescence imaging of HNO not only in living cells, but also in living tissues using two-photon fluorescence microscopy. The phosphine-based derivative for two-photon imaging of exogenous HNO in HeLa cells following excitation at 780 nm ($\lambda$Mon = 512 nm). Despite such efforts, examples of probes for the targeted detection/scavenging of HNO in specific organelles are rather uncommon, and thus, there is ample scope for the development of such reagents. Despite its many attractions, one of the drawbacks of employing optical microscopy is that it has a practical resolution limit of around 250 nm.

Luminescence-based methods are popular and powerful tool for detecting biologically active species in living cells due to its simplicity, excellent sensitivity, and well-defined spatiotemporal resolutions. The HNO fluorescent probes developed in previous studies were mostly based on the reduction of (1) Cu(II) to Cu(I), or (2) nitroxide to hydroxylamine using HNO. These probes were shown to be capable of detecting HNO in living cells. However, it was also noted that the probes might be interfered by other biologically abundant reductants in the living cells, such as glutathione and ascorbate. Furthermore, example of a reagent that could be used for monitoring of intracellular SERCA2a activity has eluded till date. Such a reagent would have a direct relevance for developing an efficient imaging reagent for HNO and as well as inhabiting SERCA2a activity induced by HNO, which would have serious implication in clinical diagnostic field.

Therefore, there is a need to design an appropriate reagent for detection and quantification of HNO under physiological as well as in biological samples for diagnostic application has significance. Accordingly, the present invention provides a compound which allow for sensitive and selective detection of HNO.

**OBJECTIVE OF THE INVENTION:**

The main objective of the present invention is to provide a novel compound of Formula (I).

Another objective of the present invention is to provide a process for the preparation of compound of Formula (I).

Yet another objective of the present invention is to provide the compound of Formula (I) for use in the detection of HNO in biological systems.

Still another objective of the present invention is to provide a kit for the detection of HNO comprising at least the compound of Formula (I).

SUMMARY OF THE INVENTION:

Accordingly, the present invention provides a compound of Formula (I);

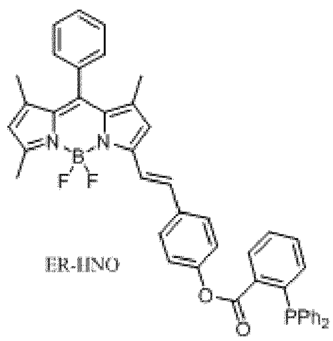

ER-HNO

Formula (I)

In another embodiment, the present invention provides a process for the preparation of compound of Formula (I) comprising the steps of:

(a) adding N, N'-Dicyclohexylcarbodiimide (DCC) to a solution of 2-(diphenylphosphino) benzoic acid in a solvent followed by stirring the mixture at temperature 0 °C for a period in the range of 1 to 2 hrs, forming a reaction mixture;

(b) adding (E)-4-(2-(5,5-difluoro-1,7,9-trimethyl-10-phenyl-5H-514,614-dipyrrolo[1,2-c:2',1'-f][1,3,2]diazaborinin-3-yl)vinyl)phenol [ER-S] and 4-Dimethylaminopyridine (DMAP) to the reaction mixture of step (a) at a temperature in the range of 25 to 30 °C for a period in the range of 6 to 7 hrs.

In preferred embodiment, the solvent used in step (a) is dichloromethane ($CH_2Cl_2$).
In another preferred embodiment, said process is carried out under nitrogen atmosphere.
In yet another embodiment, the present invention provides the compound of Formula (I) for use in the detection of HNO in biological systems.
In another embodiment, the present invention provides a fluorescent OFF-ON based molecular probe ER-HNO for the specific detection of HNO under physiological condition with excellent sensitivity.
In preferred embodiment, the compound of Formula (I) is for use as imaging reagent for HNO detection in live HCT116 cells.
In the present disclosure, the compound of Formula (I) is for use as imaging reagent for HNO detection in live RAW 264.7 cells.
In one embodiment, the present invention provides an *in vitro* method of detection of HNO in biological systems using the compound of Formula (I).
In still another embodiment, the present invention provides a kit for the detection of HNO comprising at least the compound of Formula (I).

**BRIEF DESCRIPTION OF THE DRAWINGS:**

**Fig. 1:** (A) Change in the emission spectrum of ER-HNO (10 μM) in the absence and presence of different analytes (0.2 mM); (B) emission titration profile of ER-HNO (10 μM) with varying [HNO] (0-100 μM).

**Fig. 2:** (A) Change in emission intensity of ER-HNO in presence and absence of different interfering analytes; (B) effect of pH on probe ER-HNO.

**Fig. 3:** (A) Hct116 cells stained with 1 μM of *ER-HNO* in the presence of ER tracker green; (i) intensity profile of ROIs across cells: red line represents the intensity of *ER-HNO* and green line indicates the intensity for ER tracker green; (B) co-localization experiment: cells were co-stained with *ER-HNO,* ER tracker green and DAPI; $\lambda_{Ext}/\lambda_{Em}$: 530/573 nm.

**Fig. 4:** (A) protein SERCA2 monitoring experiment using western blot assay; (b) Flow cytometry experiment of ER-HNO in presence and absence HNO.

**Fig. 5:** (A-C) Confocal laser scanning microscopic (CLSM) images of RAW 264.7 cells incubated with ER-HNO (1 μM) as the control: (A) bright field; (B) dark field laser; and (C) 3D intensity plot of image (B); (D-F) CLSM images of cells incubated with ER-HNO (1 μM) for 15 min and then further exposed to AS (20 μM) for 20 min at 37 °C: (D) bright field; (E) dark field laser; (F) 3D intensity plot of image (E). $\lambda_{Ext}/\lambda_{Em}$ =530/573 nm.

**Fig. 6:** Changes in emission intensity of ER-HNO(10 μM) induced by AS(80 μM) in the presence of a large excess (0.2 mM) of other analytes like HOCl, H2O2, .OH, TBHP, $NO_3$-, NO2-, Benzoyl peroxide, $S_2$-, NO, ONOO-and ascorbate . Gray bar and black bar represent emission response of ER-HNO (10 μM) in the presence and absence of HNO (80 μM), respectively. Studies were performed in aq. PBS buffer-$CH_3CN$ (9:1, v/v; pH 7.2) medium; $\lambda_{Ext}/\lambda_{Em}$: 530/586 nm.

**Fig. 7:** Fluorescence intensity of ER-HNO at 583 nm upon addition of HNO (0-22 μM) in acetonitrile: Aq-PBS buffer (1:9, v/v) medium at pH 7.2, $\lambda_{Ext}$ = 530 nm.

**Fig. 8:** Time dependent Emission studies of ER-HNO (10 μM) in absence and presence of 100 μM HNO in aq-PBS

-acetonitrile medium (9:1, v/v) of pH 7.2 at 37°C. $\lambda_{Ext}$ = 530 nm. $\lambda_{Mon}$ = 586 nm.

**Fig. 9:** MTT assay to determine the cell viability percentage in presence of ER-HNO in RAW 264.7 cells. $IC_{50}$ was found to be 90 μM.

**Fig. 10:** Emission titration of ER-HNO with different concentrations of AS.

**Fig. 11:** Bright field & fluorescence images of *Artemia* after 24h hatching: *Artemia* exposed to 1 μM ER-HNO; 20 μM AS (Upper row indicate bright field images; lower row represent the corresponding fluorescence images).

## DETAILED DESCRIPTION OF THE INVENTION

The invention will now be described in detail in connection with certain preferred and optional embodiments, so that various aspects thereof may be more fully understood and appreciated.

The terms "compound of Formula (I)", "ER-HNO", "ER-HNO" and "Probe ER-HNO" are used interchangeably in the specification; however they have the same meaning.

In the view of above, the present invention provides a compound of Formula (I);

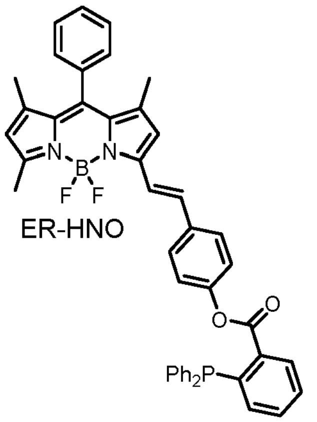

ER-HNO

Formula (I)

The IUPAC name of compound of Formula (I) is (E)-4-(2-(5,5-difluoro-1,7,9-trimethyl-10-phenyl-5H-514,614-dipyrrolo[1,2-c:2',1'-f][1,3,2]diazaborinin-3-yl)vinyl)phenyl 2-(diphenylphosphanyl)benzoate.

In another embodiment, the present invention provides a process for the preparation of compound of Formula (I) comprising the steps of:

(a) adding N, N'-Dicyclohexylcarbodiimide (DCC) to a solution of 2-(diphenylphosphino) benzoic acid in a solvent followed by stirring the mixture at a temperature 0 °C for a period in the range of 1 to 2 hrs, forming a reaction mixture;

(b) adding (E)-4-(2-(5,5-difluoro-1,7,9-trimethyl-10-phenyl-5H-514,614-dipyrrolo[1,2-c:2',1'-f][1,3,2]diazaborinin-3-yl)vinyl)phenol [ER-S] and 4-Dimethylaminopyridine (DMAP) to the reaction mixture of step (a) at a temperature in the range of 25 to 30 °C for a period in the range of 6 to 7 hrs.

In preferred embodiment, the solvent used in step (a) is dichloromethane ($CH_2Cl_2$).

In another preferred embodiment, said process is carried out under nitrogen atmosphere.

The process for the preparation of probe ER-HNO is as shown scheme 1;

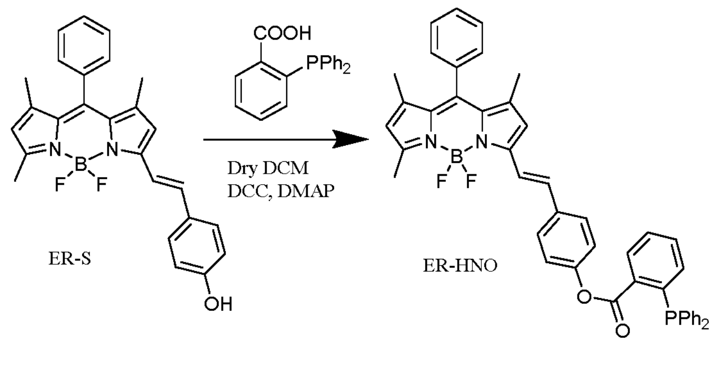


**Scheme 1:** Synthetic Methodology of Probe ER-HNO

In yet another embodiment, the present invention provides the compound of Formula (I) for use in the detection of HNO in biological systems.

In preferred embodiment, the present invention provides a fluorescent OFF-ON based molecular probe ER-HNO for the specific detection of HNO under physiological condition with excellent sensitivity.

In another preferred embodiment, the present invention provides the compound of Formula (I) for use as imaging reagent for HNO detection in live HCT116 cells.

In the present disclosure, the compound of Formula (I) is for use as imaging reagent for HNO detection in live RAW 264.7 cells.

The compound of Formula (I) exhibits high selectivity, good sensitivity and low cytotoxicity in the detection of HNO. Besides, the compound of Formula (I) shows perfect endoplasmic localization in living cells. It is observed that the compound of Formula (I) can detect HNO without interference from other analytes.

In one embodiment, the present invention provides an *in vitro* method of detection of HNO in biological systems using compound of Formula (I).

The spectroscopic properties of ER-HNO in aq. PBS buffer medium under physiological pH medium. No significant difference is observed in the electronic spectra of ER-HNO and ER-S. In order to check the response of ER-HNO probe (10 $\mu$M) with in presence of 20 mole equivalent of various ROS and RNS (HNO, HOCl, $H_2O_2$, -OH, TBHP, $NO_3^-$, $NO_2^-$, benzoyl peroxide, $S^{2-}$ and ascorbate) emission spectra are recorded in aq. PBS buffer medium are shown in Fig. 1. From fig. 1 it is observed that there is no change in emission intensity in presence of HOCl, $H_2O_2$, -OH, TBHP, $NO_3^-$, $NO_2^-$, benzoyl peroxide, $S_2^-$, Ascorbate, except HNO. A turn ON emission response is observed for probe ER-HNO, only in presence of HNO with $\lambda_{Ems}^{Max}$=580 nm ($\lambda_{Ext}$=530 nm). Moreover, no significant changes are observed in presence of different anionic, cationic as well as biothiols under identical experimental condition. The effects of pH on emission spectrum of probe ER-HNO is also investigated. The emission intensity of the probe remains unaltered within the pH range 4-10. Therefore, to explore the bio analytical application, all the studies are carried out at physiological pH medium.

The probe ER-HNO itself shows a weak emission spectrum ($\Phi$= 0.002, for $\lambda_{Ext}$= 530 nm) in aq-PBS buffer medium. An enhancement in emission signal are observed at $\lambda_{Max}$= 586 nm ($\Phi$= 0.105, for $\lambda_{Ext}$= 530 nm) with gradual increasing [HNO] (Fig. 1B). A good linearity is observed between emission intensity and concentration of HNO in the range of 2-15 uM, with a lower detection limit of 2$\mu$M. All these results demonstrate that probe ER-HNO detect trace amount of HNO quantitatively with high sensitivity under physiological pH in aq. PBS buffer medium.

The interferences studies also confirmed the emission response of ER-HNO in the presence of HNO, remain unchanged even in presence of excess amount of other analytes, used for the studies (Fig 2). This conformed that the reagent ER-HNO could be used as a switch ON fluorescence probe for the specific detection of HNO in aq. PBS medium under physiological pH.

An MTT assay confirmed the non-toxic nature of probe ER-HNO towards live Hct116. Therefore, ER-HNO is tested as an imaging reagent for the detection of HNO in Hct116 colon cancer. The cells are incubated with probe ER-HNO (1 $\mu$M) for 15min at 37°C in acetonitrile-PBS buffer (0.2:99.8, v/v) at pH 7.2 and no intracellular fluorescence is observed. CLSM images for cells that are further incubated with HNO (20 $\mu$M) showed a strong intracellular fluorescence (Fig. 3). The results shown in Fig. 3 clearly show that the reagent ER-HNO could be used as an imaging reagent for the detection of HNO uptake in live HCT116 cells.

From CLSM images as shown in Fig. 3 it is observed that this reagent ER-HNO as well as the product (ER-S) are preferentially localized in the lipid dense region, Endoplasmic Reticulum (ER) of Hct116 cells, the results of the co-localization studies with ER-tracker green (ER-specific reagent) and DAPI (nuclei specific reagent, with little or no

cytoplasmic labelling) as well as the high Pearson's co-localization coefficient of 0.975 for this co-localization study confirmed this (Fig. 3). Thus, this reagent offered an option of mapping HNO in the ER region of Hct116 cells.

The SERCA2 is monitored using ER-HNO using Western blot experiment. The Fig. 4 results confirmed that with using ER-HNO it is possible to monitor the protein activity. Moreover, the reagent ER-HNO could also successfully counteract the stabilized expression of SERCA2 to at least 50%. DCFDA staining of HCT116 cells too proved that RNS induced by Angeli's Salt is reduced when cells were treated with compound "ER-HNO". This indicate that the reagent is capable of regulating the RNS activity in the living cells (Fig. 4B).

The toxicity of the probe molecule toward live RAW 264.7 macrophages cells and cytotoxicity assays confirmed the low toxicity of the probe. The results encouraged to explore the use of the probe as an imaging reagent for the intracellular detection of HNO in these macrophage cells. Cells in DMEM culture media are incubated with ER-HNO (1.0 $\mu$M) for 15 min at 37 °C and subsequently viewed under confocal laser scanning microscope (CLSM) using a 530 nm laser as an excitation source. No intracellular fluorescence is observed until these pretreated cells are also exposed to AS, then a strong intracellular fluorescence was observed under CLSM (Figure 5).

As for the CLSM experiments, RAW macrophage cells incubated with ER-HNO (1 $\mu$M) for 15 min at 37 °C in acetonitrile-PBS buffer (0.2:99.8, v/v) at pH 7.2 displayed no intracellular fluorescence. In contrast, cells incubated with ER-HNO (1 $\mu$M) and then treated with Angeli's salt ($Na_2N_2O_3$) for 30 min, showed strong intracellular fluorescence (Figure 6). The feasibility of the reagent for biological in vivo imaging is confirmed by imaging the digestive system of the small brine shrimp, Artemia nauplii, a small marine invertebrate as the model.

In still another embodiment, the present invention provides a kit for the detection of HNO comprising at least compound of Formula (I).

The following examples, which include preferred embodiments, will serve to illustrate the practice of this invention, it is being understood that the particulars shown are by way of example and for purpose of illustrative discussion of preferred embodiments of the invention.

**Examples** Following examples are given by way of illustration therefore should not be construed to limit the scope of the invention.

**Example 1:**

**A. Synthesis of (E)-4-(2-(5,5-difluoro-1,7,9-trimethyl-10-phenyl-5H-514,614-dipyrrolo [1,2-c:2',1'-f][1,3,2]diazaborinin-3-yl)vinyl)phenyl 2-(diphenylphosphanyl)benzoate (ER-HNO):**

Under $N_2$ atmosphere, to a solution of 2-(diphenylphosphino) benzoic acid (100 mg, 0.32 mmol) in dry $CH_2Cl_2$, DCC (80 mg, 0.39 mmol) was added and stirred at 0°C for 2 hr. To this, Compound ER-S (150 mg, 0.35 mmol) and DMAP (20 mg, 0.16 mmol) were added and it was stirred at 27°C for 6 hr. Reaction was monitored by TLC. Then the mixture was concentrated under vacuum, and the crude product was purified by silica gel column chromatography by using 5% ethyl acetate in PET ether medium to give the compound ER-HNO. Yield 38%. $^1$H NMR ($CDCl_3$, 400 MHz, $\delta$ ppm): 7.73 (2H, d, *J* 6.7), 7.68 (5H, dd, *J* 12.4, 7.4), 7.63 (2H, d, *J* 5.4), 7.56 (3H, d, *J* 8.5), 7.51 (5H, t, *J* 8.6), 7.47 (5H, d, *J* 5.6), 7.32 (2H, d, *J* 4.9), 7.19 (1H, d, *J* 16.3), 6.92 (1H, d, *J*8.3), 6.60 (1H, s), 6.03 (1H, s), 2.62 (3H, s), 1.44 (3H, s), 1.41 (3H,s). $^{13}$C NMR ($CDCl_3$, 125 MHz): 190.99, 164.54, 155.27, 155.06, 153.99,153.80, 142.51, 141.53, 137.30, 134.76, 134.53,134.15, 133.94, 133.68, 133.48, 133.16,132.88, 132.35, 131.94, 131.85, 131.75, 131.44, 131.11, 130.98, 128.91, 128.67, 128.60, 128.48,128.41, 122.42, 116.15, 25.98.

**B. General experimental procedure for UV-Vis and Fluorescence studies:**

Stock solution of probe ER-HNO ($1 \times 10^4$M) was prepared in HPLC grade acetonitrile. All the analytes stock solution ($1 \times 10^{-2}$M) was prepared in aqueous HEPES buffer (10 mM) medium at pH 7.2. 500 $\mu$L of this stock solution of probe ER-HNO was added to 4.5 ml of PBS (10 mM) aqueous buffer medium having solution pH 7.2 to make the effective ER-HNO concentration of ($1 \times 10^{-5}$) M. This solution was used for all the photophysical studies. All the photophysical studies were performed in aq. PBS: $CH_3CN$ medium (9:1, v/v) at pH 7.2. All emission studies were done using $\lambda_{Ext}$ = 530 nm with an emission slit width of 2/2 nm, unless and otherwise mentioned.

**C. General procedure for confocal imaging studies:**

For confocal studies, Hct116 cells ($3 \times 10^5$) were seeded on cover slips placed in 6 well plates. After 24 hours, Hct116 cells were treated with ER-HNO (1 $\mu$M) for 30 minutes at 37°C in a 5% $CO_2$ air environment. Cells were then washed thrice with Phosphate Buffer (IX PBS) and fixed with 4% PFA for 20 minutes and washed again with IX PBS. Nail paint was used to seal the cover slips mounted on the glass slides for each well plates. Again Hct116 cells were pre-treated with ER-HNO (1 $\mu$M) for 15 minutes. Then Hct116 cells were further exposed to Angeli's salt (HNO donor),

then cells were washed thrice with media and followed by incubation with ER-HNO (1 μM) for another 15 minutes under same conditions. Cells were again washed with IX PBS buffer and fixed with 4% PFA for 20 minutes and washed again with IX PBS. Confocal laser scanning microscopic (CLSM) images were acquired in Olympus Fluoview Microscope with $\lambda_{Ext}/\lambda_{Mon}$ = 530/573 nm.

**D. Confocal Microscopy Experiments:**

RAW 264.7 cells were seeded on cover slips placed in 6 well plates. After 24 hours cells washed with DMEM medium then cells were treated with ER-HNO (1 μM) for 25 minutes. Cells were then washed thrice with culture medium and further treated with Angeli's salt (HNO donor) of different concentration for 30 min. After that cells were washed again with Phosphate Buffer Saline (3X PBS). Then cells were fixed with 4% PFA for 10 min. Again cells were washed thrice and then cover slips mounted using mounting medium. Nail paint was used to seal the coverslips mounted on the glass slides. Images were acquired by using Olympus FluoView FV-1000 Confocal Microscope.

**E. Western Blot:**

HCT116 cells were cultured in DMEM medium containing 10% Fetal Bovine Serum (FBS) supplemented with 100 Units of Penicillin Streptomycin antibiotics. Cells were treated with Angeli's salt 10 μM, ER-HNO 30 μM or both for 24 hrs. Prior to the treatment processes, DMEM medium was treated with N-Ethylmalemide (NEM) 1 mM and this medium was used to culture the cells. Post 24 hrs cells harvested and whole cell lysate proteins extracted. 40 μg of the proteins were resolved in SDS PAGE gel and transferred into PVDF membrane using Western Blot methods. The blot was over-night blocked with 5% Bovine Serum Albumin (BSA) at 4°C. 1:4000 dilution of SERCA2 antibody was to probe the blot for 2 hrs at roomtemperature (RT). After three washes with IX TBST, 1:4000 dilution of anti-rabbit HRP conjugated antibody was used to probe the blot for 1 hr at RT. After three washes with IX TBST, the blot was developed using auto-radiography in X-ray film.

**F. Flow Cytometry:**

For DCFDA staining, HCT116 cells were cultured in DMEM medium containing 5 mM glucose supplemented with 10 % FBS and 100 Units of antibiotics. Prior to the treatment processes, DMEM medium with 1 % FBS was treated with N-Ethylmalemide (NEM) 1 mM and this medium was used to culture the cells further. Cells were treated with Angeli's salt 10 μM, L 30 μM or both for 2 hrs. After treatment cells were harvested by trypsinization and washed with complete DMEM media by centrifugation. Pellet was suspended in 1 ml of PBS buffer. 10 μM DCFDA (Sigma) was added to cells followed by incubation at 37°C, 5% CO2 incubator for 45 mins. DCFDA stained cells were analyzed in BD FACS Canto II using FITC channel. For Annexin V staining cultured HCT116 cells were harvested after trypsinization and washed with IX PBS buffer. 500 μl of Annexin V binding buffer was used to re-suspend the cells. 5 μl of Annexin-V FITC antibody added to the re-suspended cell and incubated in dark for 5 mins. Antibody stained cells were then analyzed using BD Calibur FACs in FITC channel. The flow cytometry experiment confirmed that probe ER-HNO is capable of reducing RNS & ROS level induced by HNO [Fig. 5 (b)].

**G. Cell-Culture and Microscopy Experiments:**

RAW 264.7 cells were seeded on Coverslips (22 mm × 22 mm, 170 ± 5 μm square Cover glasses) placed in 6-well plates in DMEM culture medium containing (10% FBS and 1% penicillin-streptomycin) for 24 h at 37 °C, 5% CO2. After 24 h when 70% confluency was achieved the cells were washed with DMEM culture medium then cells were treated with ER-HNO probe (1 μM) for 30 min. Cells were then washed thrice with culture medium and further treated with different Angeli's salt (AS) for 30 min. Then cells were washed again with phosphate buffer saline (3 × PBS). After carrying out the Live cell uptake of the ER-HNO probe and the small molecule, the cells were fixed with 4% PFA for 15 min and then washed thrice with PBS and two times and then the coverslips were mounted using the Mounting medium (Vectashield h-1000). The coverslips were then sealed using nail varnish, and the sample was then imaged. Because SIM relies on the cell morphology, the cells were examined with a light microscope and then imaged using SIM.

**H. MTT assay:**

The *in vitro* cytotoxicity of ER-HNO on RAW 264.7 cells were determined by conventional MTT (3-(4, 5-Dimethylthiazol-2-yl)-2, 5-diphenyltetrazolium bromide) assay. RAW 264.7 cells (5 × $10^3$) were seeded in each well of a 96 well plate and cultured in a 37°C incubator supplied with 5% CO2. Cells were maintained in DMEM medium, supplemented with 10% Foetal Bovine Serum and 100 Units of Penicillin Streptomycin antibiotics. After 24 hours the cells were treated

with different concentrations of the ER-HNO in triplicates for 24 hours. After the treatment, cells were added with 0.5 μg/ml of MTT reagent. The plate was then incubated for 4 hours at 37°C. 100 μl of Isopropyl Alcohol was added to each well. Optical density was measured at 570 nm using Multiskan Go (Thermo Scientific) to find the concentration of the cell inhibition. IC50 value has been calculated to be 90 μM.

The formula used for the calculation of the MTT assay for evaluation of the cell viability was as follows:

$$\text{Cell viability (\%)} = (\text{Means of absorbance value of treated group/ Means of absorbance value of untreated control}) \times 100.$$

**I. Artemia culture and imaging: Experimental details:**

Imaging of *Artemia* nauplii by luminescence was performed using a fluorescence microscope with a 20 ×, 0.4 NA microscope objective (Olympus), coupled to an intensified CCD camera. The *Artemia* nauplii were imaged in both bright-field and epifluorescence geometries. The latter was enabled by a fluorescence filter set. The illumination intensity was about 10 W/mm$^2$. *Artemia* nauplii incubated by ER-HNO (1 μM) for 30 minutes showed no fluorescence, however on exposure to HNO, strong fluorescence was observed by fluorescence microscopy (Figure 12).

**Advantages of the invention:**

1. The reagent of the present invention specifically detects HNO over other reactive nitrogen species at neutral pH and at physiological condition.

2. The reagent of the present invention could be used to detect HNO present in the ER part of Hct116 colon cancer cells.

3. It could be used to monitor the SERCA2a antibody which plays a critical role in initiating cardiac contraction and relaxation in our body.

4. The reagent of the present invention can be utilized to detect HNO with lower detection limit.

**Claims**

**1.** A compound of Formula (I)

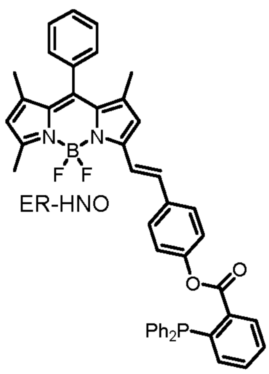

ER-HNO

Formula (I)

**2.** The compound, according to claim 1, for use in the detection of HNO in biological systems.

**3.** The compound, according to claim 1, for use as imaging reagent for HNO detection in live HCT116 cells.

**4.** A process for the preparation of compound of Formula (I) according to claim 1, comprising the steps of:

(a) adding N, N'-Dicyclohexylcarbodiimide (DCC) to a solution of 2-(diphenylphosphino) benzoic acid in a solvent followed by stirring the mixture at temperature 0°C for a period in the range of 1 to 2 hrs, forming a reaction mixture;
(b) adding (E)-4-(2-(5,5-difluoro-1,7,9-trimethyl-10-phenyl-5H-514,614-dipyrrolo[1,2-c:2',1'-f][1,3,2]diazaborinin-3-yl)vinyl)phenol [ER-S] and 4-Dimethylaminopyridine (DMAP) to the reaction mixture of step (a) at a temperature in the range of 25 to 30°C for a period in the range of 6 to 7 hrs.

**5.** The process, according to claim 4, wherein the solvent used in step (a) is dichloromethane ($CH_2Cl_2$).

**6.** The process, according to claim 4, wherein said process is carried out under nitrogen atmosphere.

**7.** A kit for the detection of HNO comprising at least the compound of Formula (I), according to claim 1.

**8.** An *in vitro* method of detection of HNO in biological systems using the compound of Formula (I), according to claim 1.

## Patentansprüche

**1.** Verbindung der Formel (I)

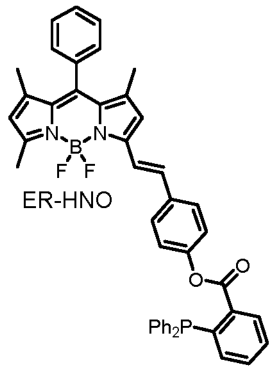

Formel (I)

**2.** Verbindung nach Anspruch 1 zur Verwendung beim Nachweis von HNO in biologischen Systemen.

**3.** Verbindung nach Anspruch 1 zur Verwendung als bildgebendes Reagenz zum Nachweis von HNO in lebenden HCT116-Zellen.

**4.** Methode zur Herstellung der Verbindung der Formel (I) nach Anspruch 1, umfassend die folgenden Schritte:

(a) Zugeben von N, N'-Dicyclohexylcarbodiimid (DCC) zu einer Lösung von 2-(Diphenylphosphino) benzoesäure in einem Lösungsmittel, gefolgt von Rühren des Gemisches bei einer Temperatur von 0 °C für einen Zeitraum im Bereich von 1 bis 2 Stunden unter Bildung eines Reaktionsgemisches;
(b) Zugeben von (E)-4-(2-(5,5-Difluor-1,7,9-trimethyl-10-phenyl-5H-514,614-dipyrrolo[1,2-c:2',1'-f][1,3,2]diazaborinin-3-yl)vinyl)phenol [ER-S] und 4-Dimethylaminopyridin (DMAP) zu dem Reaktionsgemisch aus Schritt (a) bei einer Temperatur im Bereich von 25 bis 30 °C für einen Zeitraum im Bereich von 6 bis 7 Stunden.

**5.** Methode nach Anspruch 4, wobei das in Schritt (a) verwendete Lösungsmittel Dichlormethan ($CH_2Cl_2$) ist.

**6.** Methode nach Anspruch 4, wobei die Methode unter Stickstoffatmosphäre durchgeführt wird.

7. Kit zum Nachweis von HNO, umfassend mindestens die Verbindung der Formel (I) nach Anspruch 1.

8. *In-vitro-Methode* zum Nachweis von HNO in biologischen Systemen unter Verwendung der Verbindung der Formel (I) nach Anspruch 1.

**Revendications**

1. Un composé de Formule (I),

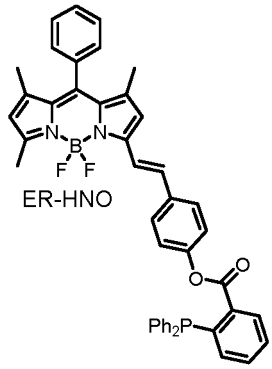

ER-HNO

Formule (I)

2. Le composé, selon la revendication 1, pour une utilisation dans la détection de HNO dans des systèmes biologiques.

3. Le composé, selon la revendication 1, pour une utilisation comme réactif d'imagerie pour la détection de HNO dans des cellules HCT116 vivantes.

4. Un procédé pour la préparation de composé de Formule (I) selon la revendication 1, comprenant les étapes suivantes:

(a) ajouter N, N'-Dicyclohexylcarbodiimide (DCC) à une solution de 2-(diphenylphosphino) acide benzoïque dans un solvant puis à agiter le mélange à une température de 0 °C pendant une durée de 1 à 2 heures, en formant ainsi un mélange réactif;
(b) ajouter (E)-4-(2-(5,5-difluoro-1,7,9-trimethyl-10-phenyl-5H-514,614-dipyrrolo[1,2-c:2',1'-f][1,3,2]diazaborinin-3-yl)vinyl)phenol [ER-S] et 4-Dimethylaminopyridine (DMAP) au mélange réactif de l'étape (a) à une température de 25 à 30 °C pendant une durée de 6 à 7 heures.

5. Le procédé, selon la revendication 4, dans lequel le solvant utilisé dans l'étape (a) est le dichlorométhane ($CH_2Cl_2$).

6. Le procédé, selon la revendication 4, dans lequel ledit procédé est exécuté sous atmosphère d'azote.

7. Un kit pour la détection de HNO comprenant au moins le composé de Formule (I), selon la revendication 1.

8. Un procédé *in vitro* de détection de HNO dans des systèmes biologiques utilisant le composé de Formule (I), selon la revendication 1.

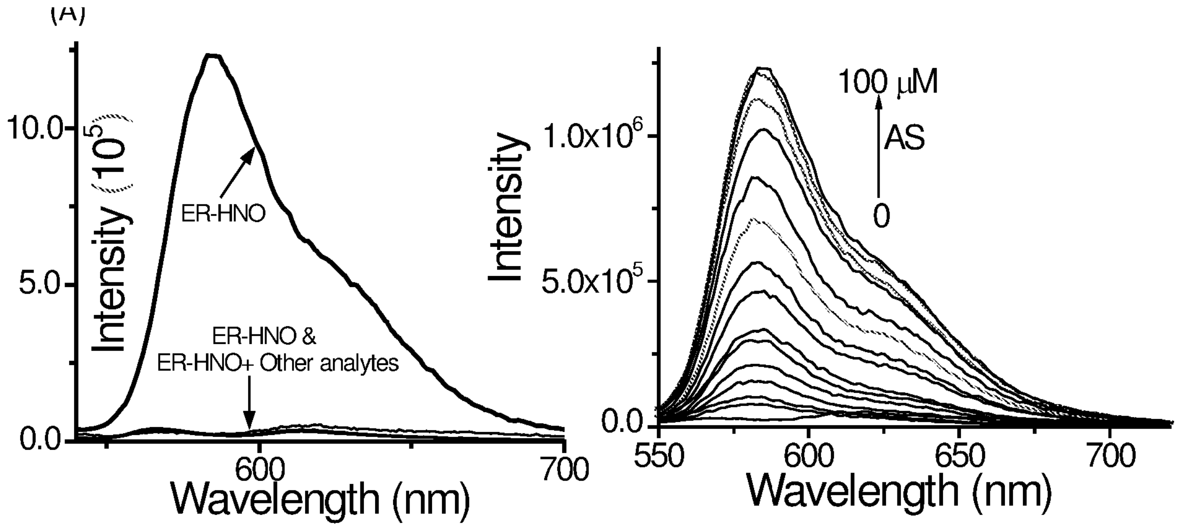
(A)
Intensity (10^5)
10.0
5.0
0.0
ER-HNO
ER-HNO &
ER-HNO+ Other analytes
600
700
Wavelength (nm)
Intensity
1.0x10^6
5.0x10^5
0.0
100 μM
AS
0
550
600
650
700
Wavelength (nm)

Fig. 1

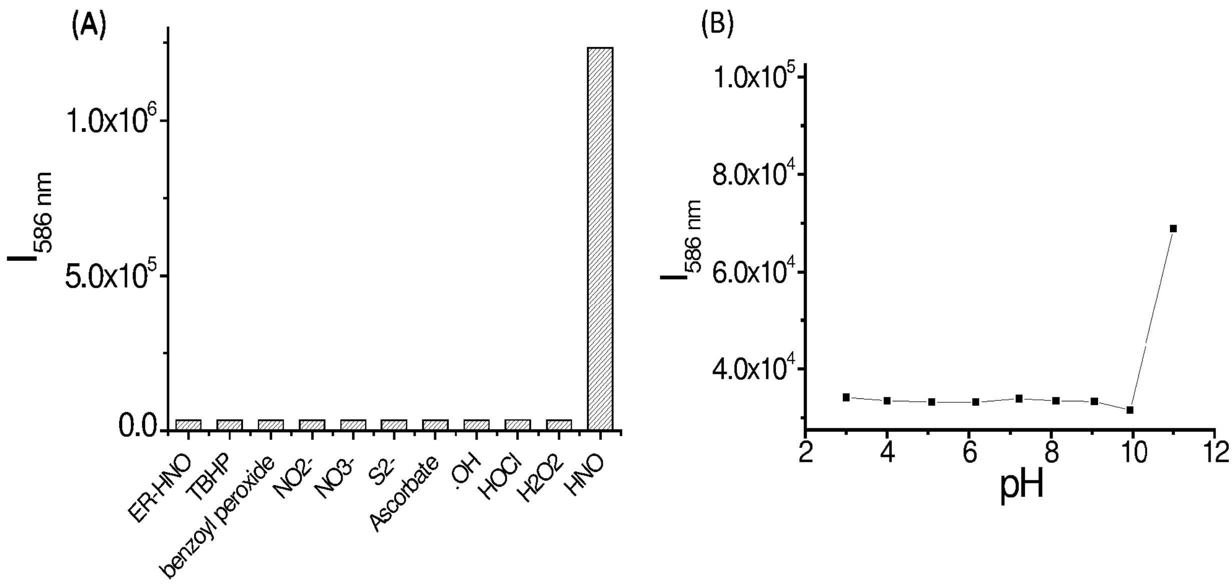
(A)
I586 nm
1.0x10^6
5.0x10^5
0.0
ER-HNO
TBHP
benzoyl peroxide
NO2-
NO3-
S2-
Ascorbate
.OH
HOCl
H2O2
HNO
(B)
I586 nm
1.0x10^5
8.0x10^4
6.0x10^4
4.0x10^4
2
4
6
8
10
12
pH

Fig. 2

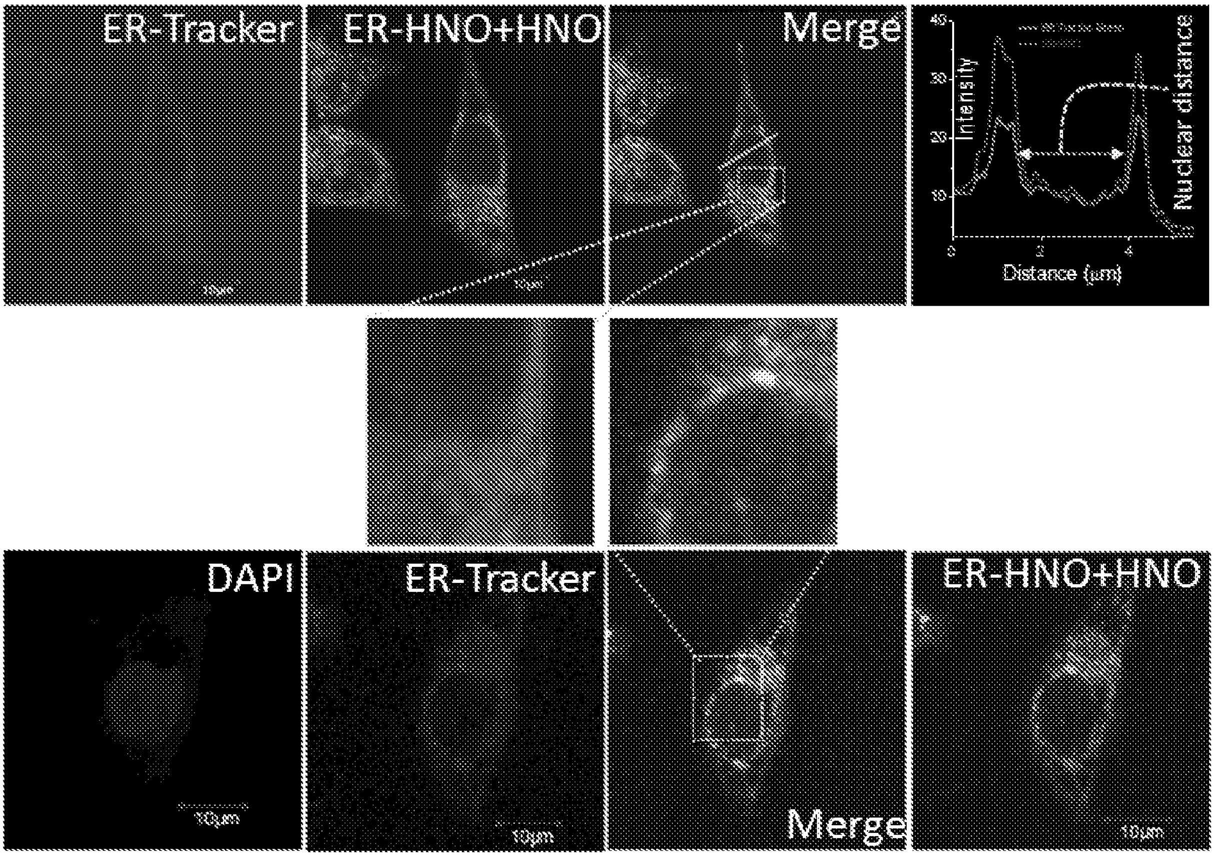
ER-Tracker
ER-HNO+HNO
Merge
Intensity
Nuclear distance
Distance (μm)
DAPI
ER-Tracker
Merge
ER-HNO+HNO
10μm

Fig. 3

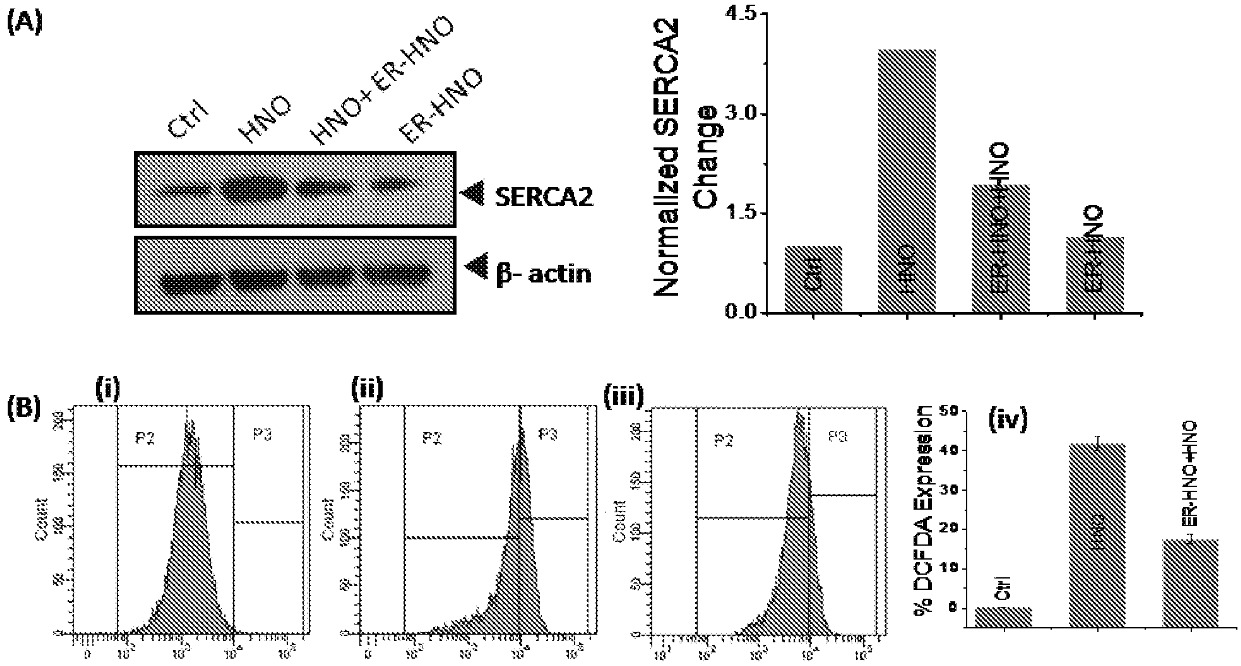
(A)
Ctrl
HNO
HNO+ER-HNO
ER-HNO
SERCA2
β- actin
Normalized SERCA2 Change
4.5
3.0
1.5
0.0
Ctrl
HNO
ER-HNO+HNO
ER-HNO
(B)
(i)
(ii)
(iii)
(iv)
P2
P3
Count
% DCFDA Expression
50
40
30
20
10
0
Ctrl
HNO
ER-HNO+HNO

Fig. 4

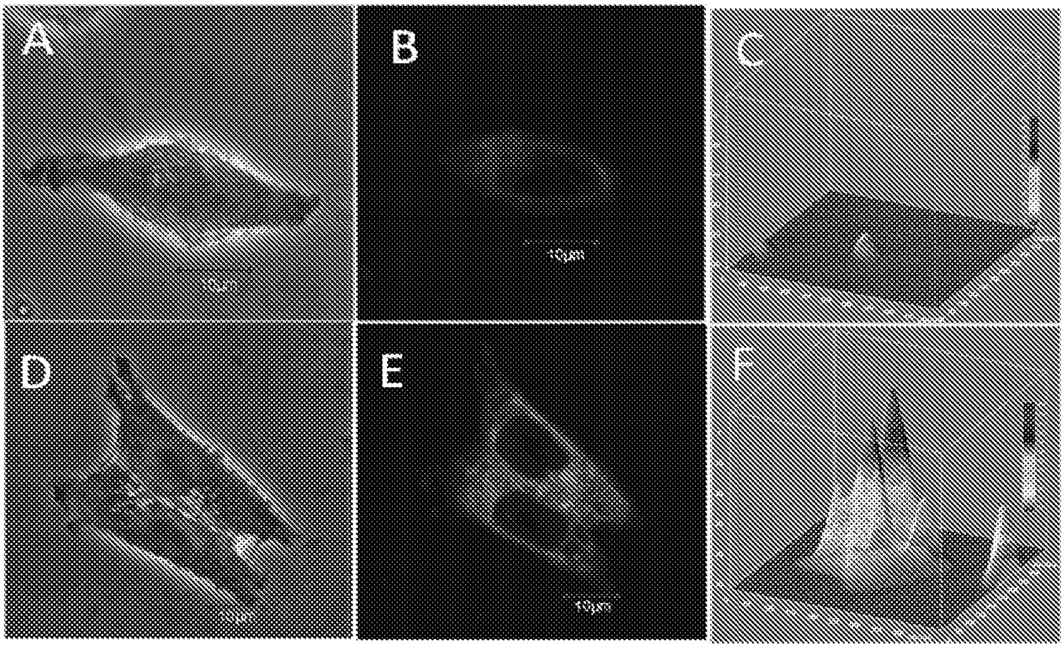
A
B
C
D
E
F
10μm
10μm
10μm

Fig. 5

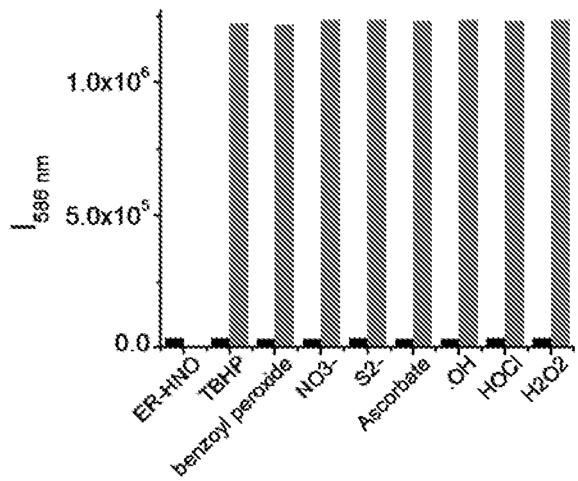
1.0x10^6
5.0x10^5
0.0
I586 nm
ER-HNO
TBHP
benzoyl peroxide
NO3-
S2-
Ascorbate
.OH
HOCl
H2O2

Fig. 6

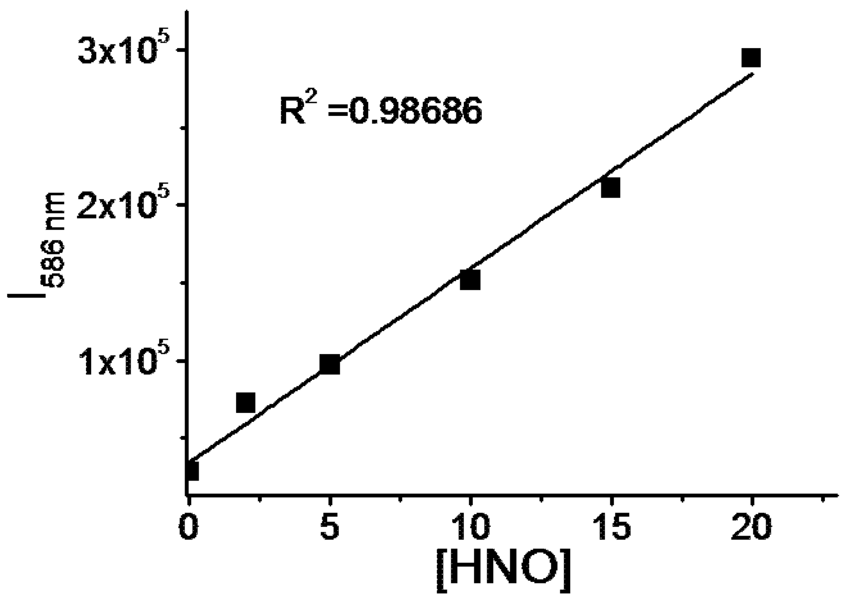
3x10^5
2x10^5
1x10^5
R^2 =0.98686
I586 nm
0
5
10
15
20
[HNO]

Fig. 7

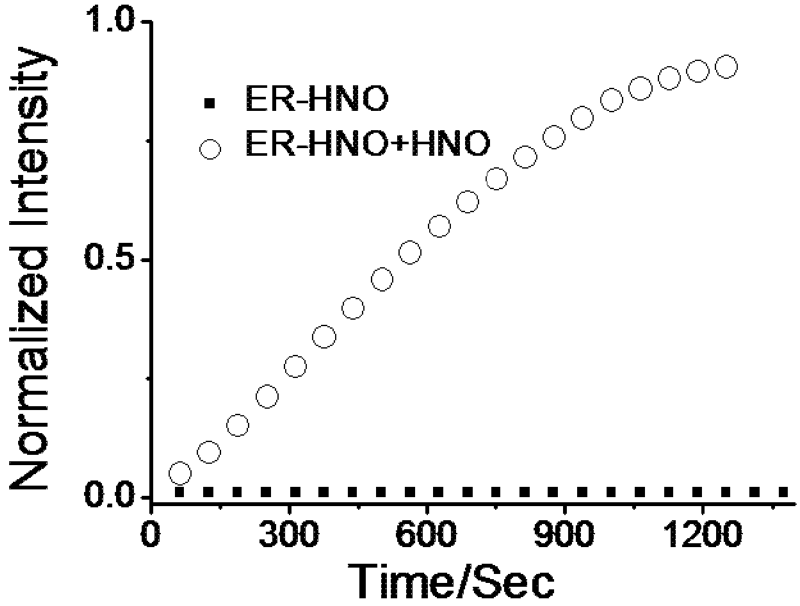

1.0
0.5
0.0
Normalized Intensity
ER-HNO
ER-HNO+HNO
0
300
600
900
1200
Time/Sec


Fig. 8

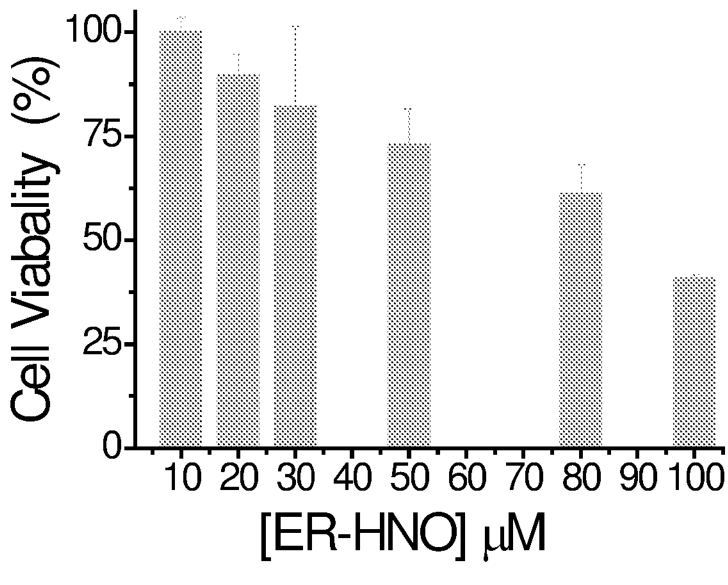

100
75
50
25
0
Cell Viabality (%)
10 20 30 40 50 60 70 80 90 100
[ER-HNO] μM


Fig. 9

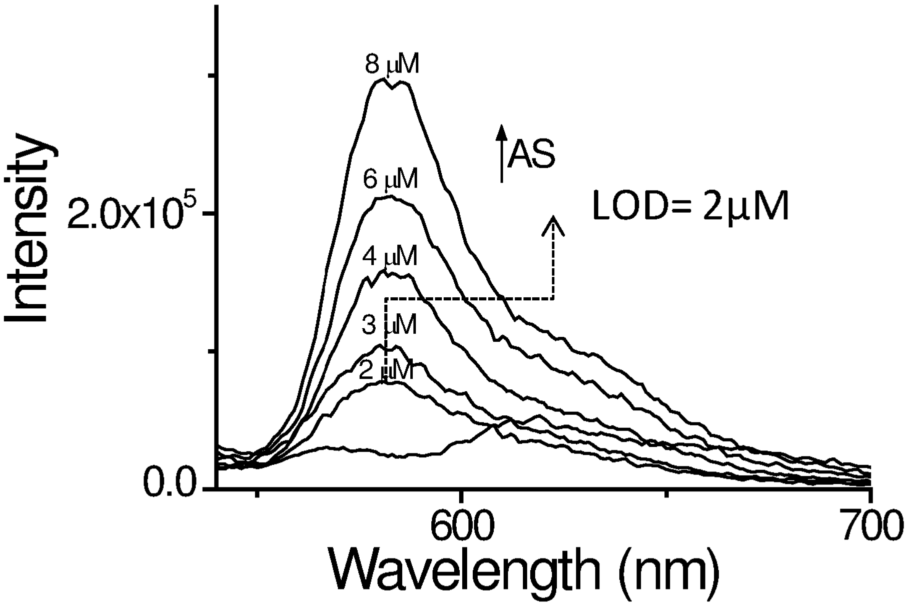
Intensity
2.0x10^5
0.0
8 µM
6 µM
4 µM
3 µM
2 µM
AS
LOD= 2µM
600
700
Wavelength (nm)

Fig. 10

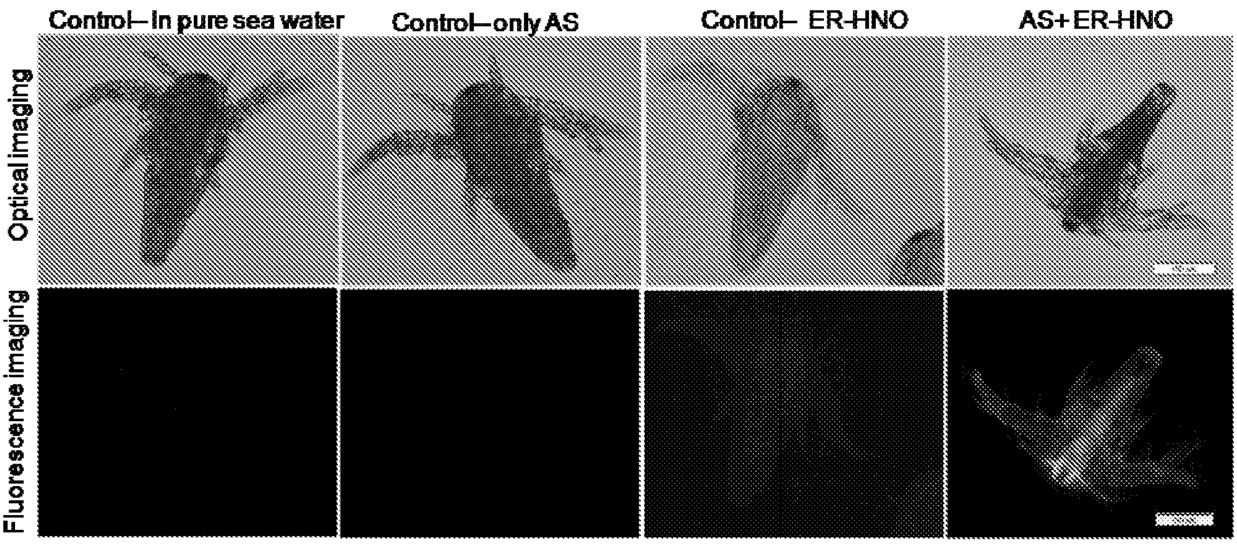
Control– In pure sea water
Control–only AS
Control– ER-HNO
AS+ ER-HNO
Optical imaging
Fluorescence imaging

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *J. Am. Chem. Soc,* 2011, vol. 133 (30), 11675-11685
- **B DONG.** Development of green to near-infrared turn-on fluorescent probes for the multicolour imaging of nitroxyl in living systems. *J. Mater. Chem. B,* 2016, vol. 4, 1263-1269
- **FIROJ ALI et al.** A fluorescent probe for specific detection of cysteine in the lipid dense region of cells. *Chem. Commun.,* 2015, vol. 51, 16932-16935
- **LIU PING et al.** A Near-Infrared Fluorescent Probe for Detection of Nitroxyl in Living Cells. *Chinese Journal of Analytical Chemistry,* 2015, vol. 43 (12), 1829-1836
- **KN BOBBA et al.** Resorufin based fluorescence 'turn-on' chemodosimeter probe for nitroxyl (HNO). *RSC Adv.,* 2015, vol. 5, 84543-84546
- **P LIU et al.** A near-infrared fluorescent probe for the selective detection of HNO in living cells and in vivo. *Analyst,* 2015, vol. 140, 4576-4583
- **GJ MAO et al.** A highly sensitive and reductant-resistant fluorescent probe for nitroxyl in aqueous solution and serum. *Chem. Commun.,* 2014, vol. 50, 5790-5792
- **K ZHENG et al.** A two-photon fluorescent turn-on probe for nitroxyl (HNO) and its bioimaging application in living tissues. *Chem. Commun.,* 2015, vol. 51, 5754-5757